(19) Europäisches Patentamt
European Patent Office
Office européen des brevets

(11) EP 3 098 228 A1

(12) EUROPEAN PATENT APPLICATION

(43) Date of publication:
**30.11.2016 Bulletin 2016/48**

(21) Application number: **15305812.8**

(22) Date of filing: **28.05.2015**

(51) Int Cl.:
***C07F 11/00*** *(2006.01)*
***C07F 13/00*** *(2006.01)*
***C07F 15/00*** *(2006.01)*
***C07F 15/02*** *(2006.01)*
***C07F 19/00*** *(2006.01)*
***A61P 35/00*** *(2006.01)*
***A61K 31/663*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **CENTRE NATIONAL DE LA RECHERCHE SCIENTIFIQUE**
  **75794 Paris Cedex 16 (FR)**
- **Université de Versailles Saint-Quentin-en-Yvelines**
  **78000 Versailles (FR)**

(72) Inventors:
- **DOLBECQ-BASTIN, Anne**
  **78035 Versailles Cedex (FR)**
- **SAAD, Ali**
  **78035 Versailles Cedex (FR)**
- **MIALANE, Pierre**
  **78035 Versailles Cedex (FR)**

(74) Representative: **Nony**
**11 rue Saint-Georges**
**75009 Paris (FR)**

# (54) POLYOXOMOLYBDATE-BISPHOSPHONATE COMPLEX CONTAINING A HETEROMETALLIC ION DIFFERENT FROM MOLYBDENUM

(57) The present invention relates to a polyoxomolybdate-bisphosphonate complex containing a bisphosphonate ligand, wherein it further contains a heterometallic ion different from molybdenum, for use in treating cancer.

It further relates to novel polyoxomolybdate-bisphosphonate complexes containing a bisphosphonate ligand, wherein it further contains a heterometallic ion different from molybdenum, to their preparation process and pharmaceutical composition containing them.

EP 3 098 228 A1

Printed by Jouve, 75001 PARIS (FR)

## Description

The present invention is generally dedicated to compounds for use for treating cancer.

The present invention more particularly concerns polyoxomolybdate-bisphosphonate complexes containing a biologically active bisphosphonate ligand, further containing a heterometallic ion, different from molybdenum, for use in treating cancer.

Polyoxometalates (POMs) constitute a large family of anionic metal oxide clusters of d-block transition metals in high oxidation states ($W^{VI}$, $Mo^{V,VI}$, $V^{IV,V}$) and have a wide range of magnetic, redox, and catalytic properties. Biological applications, in particular anti-cancer properties, have also been reported as for example in B. Hasenknopf, "polyoxometalates: introduction to a class of inorganic compounds and their biomedical applications", Frontiers in Bioscience 10, 275-287, January 1, 2005**.**

Bisphosphonates (BPs), with the general formula $H_2O_3PC(OH)(R)PO_3H_2$, are one such class of bioactive molecules that are of interest in the development of hybrid species that might have both redox and a well as more conventional activity, as enzyme inhibitors. Bisphosphonates function by inhibiting the enzyme farnesyl diphosphate synthase (FPPS), which is involved in protein prenylation and cell signaling, and have been used for almost 40 years to treat bone resorption diseases. In addition, some bisphosphonates also have activity against parasitic protozoa, as well as tumor cells. In addition, bisphosphonates have very recently been found in some systems to inactivate human epidermal growth factor receptors to exert anti-tumor activity, for example in T. Yuen, et al., Proc. Natl. Acad. Sci. USA 2014, 11, 17989 and in A. Stachnik et al., Proc. Natl. Acad. Sci. USA 2014. 111, 17995. They are known to activate human gamma-delta T cells to kill tumor cells; they switch macrophages from a tumor promoting to tumor killing phenotype and they inhibit angiogenesis. It has also been demonstrated that bisphosphonates are able to block ras signaling pathways.

Once deprotonated, BPs act as multidentate ligands and can form stable POM complexes. A broad range of POM/BP hybrids were previously reported for example in A. Banerjee et al. Chem. Soc. Rev. 2012, 41, 7590, in A. Dolbecq, et al., Chem. Commun. 2012, 48, 8299 and in L. Yang et al., Cryst. Growth. Des. 2013, 13, 2540, in connection to their anti-tumor cell growth inhibition activity. In particular, the activity of Mo(VI) complexes with BPs against tumor cell lines has been studied, suggesting synergistic effect between both organic and inorganic components (H. El Moll et al., "polyoxometalales functionalized by bisphonate ligands: synthesis, structural, magnetic and spectroscopic characterizations and activity on tumor cell lines", Inorg. Chem. 2012, 51, 7921-7931).

There is a continuing need for proposing polyoxomolybdate-bisphosphonate complexes demonstrating anti-cancer activity.

There is also a need to provide the skilled person with polyoxomolybdate-bisphosphonate complexes showing synergistic activity in comparison to polyoxomolybdate complex alone and the corresponding BP alone. In other words, there is a need to propose polyoxomolybdate-bisphosphonate complexes with an activity of combined origin, i.e. metal based and also demonstrating significant effect on protein prenylation.

There is an additional need to provide the skilled person with new chemical entities increasing activity in tumor cell killing.

The present invention has for purpose to meet these needs.

Unexpectedly, the inventors have found that the addition of a heterometallic element different from molybdenum imparts interesting properties, including perhaps, targeting of isoprenoid biosynthesis by the bisphosphonate ligands.

Therefore, the present invention concerns a polyoxomolybdate-bisphosphonate complex containing a bisphosphonate ligand, wherein it further contains a heterometallic ion different from molybdenum, for use in treating cancer, said polyoxomolybdate-bisphosphonate complex having in particular the formula (I) as defined below.

The present invention moreover relates to a method of preventing, inhibiting or treating cancer, which comprises at least one step consisting in administering to a patient suffering therefrom an effective amount of a polyoxomolybdate-bisphosphonate complex containing a bisphosphonate ligand, wherein it further contains a heterometallic ion different from molybdenum, and in particular of a complex as defined in formula (I) below.

Some of said complexes of formula (1) are new and also form part of the present invention, and subfamilies (Ia) and (Ib) as defined below, which pertain to the complex of formula (I) are also encompassed within the scope of the present invention.

The present invention further relates to a process for the preparation of said complexes of formula (I) which are new, consisting in reacting

(i) a precursor selected from $Na_2MoO_4$ and $(NH_4)_6Mo_7O_{24}$ within a one-pot procedure in presence of a solvent, in particular water buffered between 3 and 8, BP, a M metal salt at a temperature ranging from 20 to 130°C, or
(ii) a $Na_2MoO_4$ precursor within a two step procedure comprising a first step using said $Na_2MoO_4$ precursor in water at pH 3, followed by a second step using the obtained $Mo_{12}(BP)_4$ in water at pH 3, a M precursor at a temperature ranging from 20 to 100°C.

The present invention also provides pharmaceutical compositions comprising at least one of said complexes of formula (I) which are new.

Due to multiple site of action of the complexes according to the present invention, it is expected to decrease the likelihood of drug resistance, which is a great advantage for anti-cancer drugs.

In the context of the present invention, the expression:

- "polyoxometalates" or "POMs" as used herein refer to negatively charged aggregates of transition metals (mainly Vanadium, Molybdenum and Tungsten) with oxygen,
- "patient" may extend to humans or mammals, such as cats or dogs.

The terms "between... and..." and "ranging from... to..." should be understood as being inclusive of the limits, unless otherwise specified.

## FIGURES

Figure 1 represents a complex of formula (Ia) as defined herein after.

Figure 2 represents an intermediate compound of complex of formula (Ib) as defined herein after.

Figure 3a represents $\chi_M T$ = f($T$) curves related to the complexes **$Mo_4Ale_2Mn$** and **$Mo_4Zol_2Mn$.** Figures b and c represent the $M$ = f($H$) curves related to the complexes **$Mo_4Ale_2Mn$** and **$Mo_4Zol_2Mn$** respectively recorded at 3, 4, 6 and 8 K.

## POLYMOLYBDATE-BISPHOSPHONATE COMPLEX

According to one aspect, a subject-matter of the present invention relates to a polyoxomolybdate-bisphosphonate complex containing a bisphosphonate ligand, wherein it further contains a heterometallic ion different from molybdenum, for use in treating cancer.

According to a further aspect, the present invention relates to a polyoxomolybdate-bisphosphonate complex containing a bisphosphonate ligand, wherein it further contains a heterometallic ion different from molybdenum, for use in treating cancer, having the following formula (I)

$$[Mo_xO_y(BP)_zM_tX_r]^{n-} \qquad (I)$$

wherein

x, y, z, t, r and n are independent integers,
$2 \leq x \leq 12$,
y > x, and y ranges from 4 to 40,
$z \leq x$, and z ranges from 2 to 8,
t < x and t ranges from 1 to 10,
r ranges from 0 to 8,
n represents an integer ranging from 2 to 16,
M represents a 3d transition metal ion selected from Mn, Fe, Cu, Zn, V and Co or a 4d or 5d transition metal like Ru and Pt,
X represents an halogen atom or a water molecule, and
BP represents a bisphosphonate, as defined herein after.

All the diversity of nuclearities and shapes of the complexes form part of the present invention. As it will be apparent in the following, depending on the process and for example the precursors, the operating conditions (particularly pH and temperature), the assembly between the precursor and the ligand(s) comes out in various forms. This has been exemplified in a recent study (Inorg. Chem. 2012, 51, 7921-7931) where it is shown that according to the pH value, three different nuclearities have been observed for the same BP: x = 1 at pH 7.5, x = 6 at pH 4.8 and x = 12 at pH 3.

According to a preferred embodiment, the present invention relates to a compound wherein M is Fe, Pt, Mn, V or Ru, in particular Mn, V and Ru and more particularly Mn.

According to a particular embodiment, the present invention relates to a polyoxomolybdate-bisphosphonate complex as defined above, for use in treating cancer, wherein the polyoxometalate-bisphosphonate complex contains $Mo_4$ or $Mo_{12}$ clusters and in particular a $Mo_4$ cluster.

According to a further particular embodiment, x=4 or 12. It may be noticed that once x is fixed, the other variable indices y, z and t naturally derive there from due to configurational constraints. With respect to the charge of the complex, n may vary between 2 and 16.

According to another particular embodiment, an additional subject-matter of the present invention is a polyoxomolybdate-bisphosphonate complex having the following formula (Ia)

$$[Mo_4O_{12}(BP)_2M]^{n-} \qquad (Ia)$$

wherein

BP is a bisphosphonate, in particular as defined hereinafter,
n represents an integer ranging from 4 to 8, and
M represents a 3d transition metal ion selected from Mn, Fe, Cu, Zn, V and Co or a 4d or 5d transition metal like Ru and Pt, and in particular M represents Mn, V or Ru, and more particularly Mn,
with the exclusion of compounds $Mo_4O_{12}Eti_2Mn$ (Mn(II)), $Mo_4O_{12}Ale_2Mn$ (Mn(II)), $Mo_4O_{12}Ale_2V$ (V(IV)), $Mo_4O_{12}Eti_2Fe$ (Fe(III)) and $Mo_4O_{12}Eti_2V$ (V(IV)).
$Mo_4O_{12}Ale_2V$ (V(IV)) and $Mo_4O_{12}Eti_2V$ (V(IV)) are disclosed in Y. Li, E.Wang et al "Two new cantilever-type polyoxometalates constructed from {Mo2O4}2+ fragments and diphosphonates" Dalton Trans 2010, 39, 1245; $Mo_4O_{12}Ale_2Mn$ (Mn(II)) in A. Dolbecq et al "Molybdenum bisphosphonates with Cr(III) or Mn(III) ions" J. Clust. Sci. 2014, 25, 795, and $Mo_4O_{12}Eti_2Fe$ (Fe(III)) and $Mo_4O_{12}Eti_2Mn$ (Mn(II)) are disclosed in Y. Zhou et al "A general synthesis approach in action: preparation and characterization of polyoxomolybdenum(VI) organophosphonates through oxidative Mo-Mo bond cleavage in {MoV2O4}" CrystEng Comm 2012, 14, 4826.

Said complex of formula (Ia) is represented in figure 1, wherein BP means bisphosphonate that may for example be alendronate or zoledronate, as explained herein after.

According to this particular embodiment, the structures are centro-symmetric and contain two $\{Mo_2O_6\}$ dimeric units having face-shared $Mo^{VI}$ octahedra bound to an octahedrally coordinated metallic ion, M, which is located on a (pseudo) inversion center. In each $Mo^{VI}$ dimer, molybdenum ions are connected to a pentadentate BP ligand via P-O-Mo and C-O-Mo bonds. The BP ligands are also bound to the central 3d transition metal ion or 4d transition metal.

According to another particular embodiment, an additional subject-matter of the present invention is a compound of
the following formula (Ib)

$$[Mo_{12}O_{32}(BP)_4M_4X_8]^{n-} \qquad (Ib)$$

wherein BP is a bisphosphonate in particular as defined hereinafter,
n represents an integer ranging from 8 to 16,
M represents a 3d transition metal ion selected from Mn, Fe, Cu, Zn, V and Co or a 4d or 5d transition metal like Ru and Pt, and in particular M represents Pt, and
X represents a halogen atom or a water molecule.
Said $Mo_{12}$ core or intermediate compound of the complex of formula (Ib) is represented in figure 2.

According to this particular embodiment, the structures may be described as the connection of four trimeric $\{Mo_3O_8\}$ units via two oxygen atoms, one from a P-O bond and one from a Mo-O bond. The heterometallic ion M is connected to the complex via chelating groups present on the R arm of the BP ligand.

It may be noticed that in some abbreviated names of complexes according to the present invention, the elements O and X of the formulae are not always represented, as it will be apparent in table 1 and in the experimental part herein after.

The heterometallic ion different from molybdenum may exist in various oxidation states which are all included in the framework of the present invention.

The oxidation state of said heterometallic ion may be determined by magnetic measurements.

For example Mn and Fe may exist in oxidation state II or III; V may exist in oxidation state IV or V; Ru preferably exists in oxidation state III.

The structure of the complexes may be determined by using single crystal X-ray diffraction, elemental analysis, IR spectrometry, magnetic susceptibility and ID and 2D solid state NMR according to methods known to the man skilled in the art.

In some cases, the structure of the complexes may be determined by solid state NMR spectrometry, as it is for example illustrated herein after for complexes with Pt.

The complexes may be in the form of variable supramolecular arrangements due to hydrogen bonding interactions depending on the nature of the organic arm of the BP ligands. All of said supramolecular arrangements also form part of the present invention.

The complex of the present invention can be prepared by conventional methods of polyoxometalate synthesis practiced by those skilled in the art. The general reaction sequences outlined below represent general methods useful for preparing the complexes of the present invention and are not meant to be limiting in scope or utility.

The synthetic routes may be different depending on the additional heterometallic element.

The complex of general formula (Ia) can be prepared according to scheme 1 below.

Scheme 1

$$Na_2MoO_4 \text{ or } (NH_4)_6Mo_7O_{24} \xrightarrow{\text{1) solvent; 2) BP; 3) M salt and 4) pH adjustment}} [Mo_4O_{12}(BP)_2M]^{n-} \text{ (Ia)}$$

The synthesis is based on the use of one precursor, for example selected from $Na_2MoO_4$, $(NH_4)_6Mo_7O_{24}$, $MoO_3$ and $Li_2MoO_4$, and in particular from $Na_2MoO_4$ and $(NH_4)_6Mo_7O_{24}$ within a one-pot procedure similar to that used for the $Cr^{III}$-containing molybdobisphosphonates as described in A. Saad et al., J. Clust. Sci. 2014, 25, 795, in the presence of a solvent, the BP, the M metal salt, in a pH ranging from 3 to 8, and in particular from 6 to 7.5, and preferably under stirring. Said reaction may be carried out at a temperature ranging from 20 to 130°C, for example during a period ranging from 5 minutes to 20 hours. Said step may be followed by a cooling at room temperature and a slow evaporation after centrifugation and/or filtration to recover a fine powder.

According to this procedure, the solvent may be water. The pH may be adjusted by the presence of a buffer, for example a $CH_3COONH_4/CH_3COOH$ buffer or a $CH_3COOLi/CH_3COOH$ or $CH_3COONa/CH_3COOH$ buffer, for example in a concentration ranging from 0.5 to 2M. The metal salt may be selected from $Mn(CH_3COO)_3.2H_2O$, $FeCl_3{\cdot}6H_2O$, $Cu(CH_3COO)_2{\cdot}H_2O$, $NaVO_3$, $VOSO_4{\cdot}5H_2O$, $RuCl_3{\cdot}xH_2O$, $PtCl_2(DMSO)_2$, $Zn(CH_3COO)_2.2H_2O$ and $Co(CH_3COO)_2.4H_2O$.

The complex of general formula (Ib) can be prepared according to scheme 2 below.

Scheme 2

$$Na_2MoO_4 \xrightarrow{\text{1) BP; 2) pH adjustment}} Mo_{12}(BP)_4 \xrightarrow[\text{3) NaCl}]{\text{1) M precursor; 2) pH adjustment}} [Mo_{12}O_{32}(BP)_4M_4X_8]^{n-}$$

(Ib)

The synthesis is based on a two step procedure.

The first step may be carried out in a solvent, such as water, in the presence of the precursor $Na_2MoO_4$, a BP, in a pH between 3 and 5, and preferably under stirring. Said first step may be carried out at a temperature ranging from 20 to 90°C for example during a period ranging from 10 minutes to 1 hour. Said step may be followed by a cooling at room temperature and a slow evaporation after centrifugation and/or filtration to recover a fine powder.

The second step may be implemented by reacting the powder as obtained in the first step, i.e. $Mo_{12}(BP)_4$, in a solvent, such as water, in the presence of the M precursor, in a pH ranging from 3 to 5, and preferably under stirring. Said second step may be carried out at a temperature ranging from 20 to 90°C, for example during a period ranging from 10 minutes to 1 hour. Said step may be followed by a cooling at room temperature and a slow evaporation after centrifugation and/or filtration to recover a fine powder.

The M precursor may be selected from $Pt(DMSO)_2Cl_2$.

The present invention further relates to a synthetic preparation process for obtaining a polyoxomolybdate which are new, consisting in reacting

(i) a precursor selected from $Na_2MoO_4$ and $(NH_4)_6Mo_7O_{24}$ within a one-pot procedure in presence of a solvent, in particular water buffered between 3 and 8, BP, a M metal salt at a temperature ranging from 20 to 130°C, or

(ii) a $Na_2MoO_4$ precursor within a two step procedure comprising a first step using said $Na_2MoO_4$ precursor in presence of a solvent, in particular water buffered between 3 and 5, followed by a second step using the obtained $Mo_{12}(BP)_4$ in presence of a solvent, in particular water buffered between 3 and 5, BP, a M precursor at a temperature

ranging from 20 to 90°C.

The availability of the precursors is discussed below. With respect to the preparation of polyoxomolybdates complexes containing $Mo^{VI}$ ions with bisphosphonates ligands, it is well known to the man skilled in the art to use a $Na_2MoO_4$ or a $(NH_4)_6Mo_7O_{24}$ precursor, which are besides commercially available. A solution of $Mo^{VI}$ ions is simply prepared by dissolving the precursor in water. The pH value has to be controlled. The BP/Mo and M/Mo ratios are crucial parameters. The order of addition of reactants plays also a role. Temperature can be increased up to 130°C in case of poorly soluble BP. Addition of extra counter-cations ($Na^+$, $Rb^+$) can favor the crystallization.

<u>BISPHOSPHONATES</u>

Bisphosphonates that may be implemented in the framework of the present invention may be selected from all the classical ones as already disclosed in the literature. Among known bisphosphonates, the following may be cited: etidronate, pamidronate, neridronate, olpadronate, alendronate, ibandronate, risedronate and zoledronate.

Bisphosphonates in the framework of the present invention may be defined as compounds of general formula
$H_2O_3PC(OH)(R)PO_3H_2$ (A)
wherein R represents

- a $(C_1\text{-}C_8)$alkyl group, optionally substituted by a NR1R2 group, or
- a

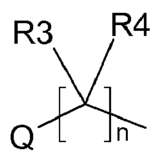

group
Wherein

n is 1, 2 or 3,
R3 and R4 independently of each other are selected from a hydrogen atom, a halogen, a $(C_1\text{-}C_4)$alkyl group and a $(C_1\text{-}C_4)$alkoxy group, and
Q represents a phenyl group or a heteroaryl group, said phenyl and heteroaryl being optionally substituted with one to three substituents chosen from a $(C_1\text{-}C_{15})$alkyl group, a halogen atom, a hydroxyl group, an OR1 group, a SRI group, a NR1R2 group or a CN group, and
R1 and R2 independently represent a hydrogen atom, a $(C_1\text{-}C_6)$alkyl or a $-(CH_2)_xX$, wherein x is 1, 2 or 3, and X has the same meaning as Q.

In the context of the present invention:

- The term "alkyl" as used herein refers to a linear or branched, saturated aliphatic hydrocarbon group. For instance a $(C_1\text{-}C_6)$alkyl group denotes a linear or branched carbon chain of 1 to 6 carbon atoms. Examples are, but are not limited to methyl, ethyl, propyl, isopropyl, butyl and methylbutyl.
- The term "heteroaryl" denotes a 5- or 6-membered aromatic ring comprising 1 or 2 heteroatoms,
- The term "heteroatom" is understood to mean nitrogen, oxygen or sulphur. Preferably, the heteroaryl comprises at least one nitrogen atom. According to a particular embodiment, the heteroaryl comprises only nitrogen as heteroatom(s). Thus, advantageously, the heteroaryl comprises from 1 to 4 nitrogen atoms, and preferably 1 or 2 nitrogen atoms.

Mention may be made of pyridine, thiazole, pyrrole, pyrazole, imidazole and triazole, and more particularly of imidazole.

In the context of the present invention, the term "heteroaryl" includes all the positional isomers.

Among the compounds of general formula (A), a first subgroup of compounds is formed from compounds for which R represents a $(C_1\text{-}C_8)$alkyl group, optionally substituted by a NR1R2 group, with R1 and R2 having the same meaning as described above.

Among the compounds of general formula (I), a second subgroup of compounds is formed from compounds

for which R represents a

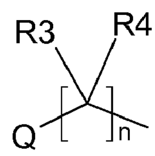

group wherein

n is 1, 2 or 3,
R3 and R4 independently of each other are selected from a hydrogen atom, a halogen, a $(C_1-C_4)$alkyl group and a $(C_1-C_4)$alkoxy group, and
Q represents a phenyl group or a heteroaryl group, said phenyl and heteroaryl being optionally substituted with one to three substituents chosen from a $(C_1-C_{15})$alkyl group, a halogen atom, a hydroxyl group, an OR1 group, a SRI group, a NR1R2 group or a CN group.

According to a particular embodiment, the bisphosphonate is defined as a compound of formula (A) wherein R represents a

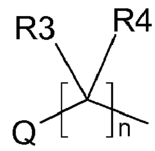

group in which n is 1, R3 and R4 are hydrogen atoms and Q is a

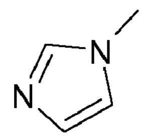

group, optionally substituted by a $(C_1-C_{15})$alkyl group.

According to an even more particular embodiment, the bisphosphonate is defined as a compound of formula (A) wherein R represents a

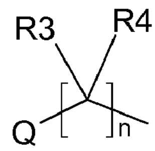

group in which n is 1, R3 and R4 are hydrogen atoms and Q is a

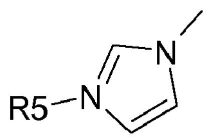

group, where R5 represents a hydrogen atom or a $(C_1-C_{15})$alkyl group. This particular subgroups encompasses compound BPH-1222 as disclosed in Yifeng Xia et al., "a combination therapy for KRAS-driven lung adenocarcinomas using lipophilic bisphosphonates and rapamycin", ScienceTranslationMedicine, Vol6, issue 263, 2014, 263ra161.

According to a further particular embodiment, the present invention is directed to a compound of formula (I) for use in treating cancer, wherein BP is a compound of formula (A) wherein R represents a $(C_1-C_8)$alkyl group, optionally substituted by a NR1R2 group or a

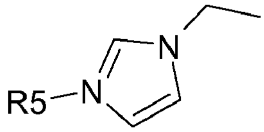

,

where R1 and R2 independently represent a hydrogen atom or a methylpyridine group, in particular a 2-methylpyridine group, and where R5 represents a hydrogen atom or a $(C_1-C_8)$alkyl group.

Structures and ligand abbreviations which may more particularly be implemented in the framework of the present invention are as follows:

| Abbreviation | R |
|---|---|
| Ale (alendronate) | 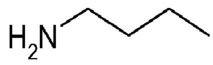 |
| Zol (zoledronate) | 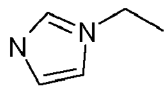 |
| $BPC_8NH_2$ | 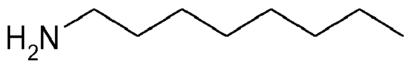 |
| AlePy | 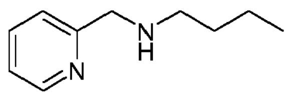 |
| $BPC_9$ | 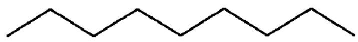 |
| BPH-1222 | 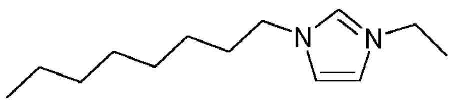 |

The present invention relates to a polyoxomolybdate-bisphosphonate complex of formula (I) as defined above, for use in treating cancer, wherein x, y, z, t, n and M are as defined above and wherein the bisphosphonate ligand is chosen among alendronate, zolendronate, BPC8NH2, AlePy, BPH-1222 and BPC9, and more particularly wherein M is Mn.

## APPLICATION

The complexes as defined in the present invention are useful in the prevention and treatment of cancer.

According to the present invention, the term "preventing" or "prevention" means to reduce the risk of onset or slow the occurrence of a given phenomenon, namely, a cancer.

As detailed hereinafter in the experimental part, some of the complexes according to the present invention were tested to attest their activity against the human breast adenocarcinoma cell line MCF-7.

Moreover, cell growth inhibition was rescued by addition of geranylgeraniol, which reverses the effects of bisphosphonates on isoprenoid biosynthesis/protein prenylation. Overall, the results indicate an important role for both the heterometallic element as well as the bisphosphonate ligand in the mechanism of action of the most active compounds of the present invention.

The following type of cancer may more particularly be treated by the complex according to the present invention: colorectal cancer, pancreatic cancer, lung cancer including non-small cell lung cancer, breast cancer, large intestine cancer, biliary tract cancer, bladder cancer, gall bladder cancer, thyroid cancer, melanoma, liver cancer, uterine/cervical cancer, oesophageal cancer, kidney cancer, ovarian cancer, prostate cancer, head and neck cancer, and stomach cancer, etc.

According to a particular embodiment, the present invention is more particularly directed to the prevention and/or treatment of lung, large intestine, biliary tract, breast and pancreatic cancer.

According to a further particular embodiment, the present invention is more particularly directed to a method

for treating patients suffering from a Ras-associated cancer, wherein said cancer is preferably associated with overactivated Ras.

In other words, in a preferred embodiment the cancer cells will harbor the ras oncogene.

According to a further more particular aspect of the present invention, it is directed to a method for treating patients suffering from a Ras-associated cancer, wherein said Ras protein is selected from HRAS, KRAS and NRAS.

The one skilled in the art may notably refer to the article of Miller et al. (2012, Frontiers in Genetics, Vol. 2, Article 100: 1-9).

According to some embodiments, the present invention relates to methods for treating patients suffering from a cancer wherein Ki-Ras comprises one or more mutations, which encompasses cancers wherein the Ki-Ras one or more mutations are present and are are selected in a group comprising $CYS^{12}$ $ASP^{12}$, $VAL^{12}$, $ALA^{12}$, $SER^{12}$, $ARG^{12}$, $PHE^{12}$, $ASP^{13}$, $CYS^{13}$, $ARG^{13}$, $SER^{13}$, $VAL^{13}$, $ALA^{13}$, $HIS^{61}$, $ARG^{61}$ and $ASP^{61}$. (See Miller et al., 2012, *Supra*).

For this purpose an effective amount of a said compound may be administered to a patient suffering from cancer.

**PREFERRED COMPLEX**

According to a preferred embodiment of the present invention, a complex according to the invention is chosen among:

- **(1) $Mo_4O_{12}Ale_2Fe$,**
- **(2) $Mo_4O_{12}(BPC_8NH_2)_2Fe$,**
- **(3) $Mo_4O_{12}(BPC_9)_2Fe$,**
- **(4) $Mo_4O_{12}Zol_2Fe$,**
- **(5) $Mo_4O_{12}Ale_2Mn$** (**Mn**(**II**)),
- **(6) $Mo_4O_{12}Zol_2Mn$ (Mn(III)),**
- **(7) $Mo_{12}O_{32}(AlePy)_4Pt_4Cl_8$,**

The following complexes may be further cited as forming part of the present invention:

- **(8) $Mo_4O_{12}Ale_2Mn$ (Mn(III)),**
- **(9) $Mo_4O_{12}Zol_2Mn$ (Mn(II)),**
- **(10) $Mo_4O_{12}(BPH\text{-}1222)_2Mn$ (Mn(II)),**
- **(11) $Mo_4O_{12}(BPH\text{-}1222)_2Mn$ (Mn(III)),**
- **(12) $Mo_4O_{12}Zol_2V$ (V(IV)), and**
- **(13) $Mo_4O_{12}Zol_2Ru$ (Ru(III)).**

The chemical structures and status of some compounds of formula (I) of the invention are illustrated in the following Table I.

**Table 1**

| Numéro | Abbreviated Formula | x, y, z, t values | Status |
|---|---|---|---|
| **1** | **$Mo_4Ale_2Fe$** | x = 4, y = 12, z = 2, t = 1 | New |
| **2** | **$Mo_4(BPC_8NH_2)_2Fe$** | x = 4, y = 12, z = 2, t = 1 | New |
| **3** | **$Mo_4(BPC_9)_2Fe$** | x = 4, y = 12, z = 2, t = 1 | New |
| **4** | **$Mo_4Zol_2Fe$** | x = 4, y = 12, z = 2, t = 1 | New |
| **5** | **$Mo_4Ale_2Mn$ (Mn(II))** | x = 4, y = 12, z = 2, t = 1 | Disclosed in A. Dolbecq et al J. Clust Sci. 2014, 25, 795 |
| **6** | **$Mo_4Zol_2Mn$ (Mn(III))** | x = 4, y = 12, z = 2, t = 1 | New |
| **7** | **$Mo_{12}(AlePy)_4Pt_4$** | x = 12, y = 32, z = 4, t = 4 | New |
| **8** | **$Mo_4Ale_2Mn$ (Mn(III))** | x = 4, y = 12, z = 2, t = 1 | New |

(continued)

| Numéro | Abbreviated Formula | x, y, z, t values | Status |
|---|---|---|---|
| **9** | **$Mo_4Zol_2Mn$ (Mn(II))** | x = 4, y = 12, z = 2, t = 1 | New |
| **10** | **$Mo_4(BPH\text{-}1222)_2Mn$ (Mn(II))** | x = 4, y = 12, z = 2, t = 1 | New |
| **11** | **$Mo_4(BPH\text{-}1222)_2Mn$ (Mn(III))** | | New |
| **12** | **$Mo_aZol_2V$ (V(IV))** | x = 4, y = 12, z = 2, t = 1 | New |
| **13** | **$Mo_4Zol_2Ru$ (Ru(III))** | x = 4, y = 12, z = 2, t = 1 | New |

Therefore, the present invention extends to complexes (1) to (4) and (6) to (13).

The examples below of compositions according to the invention are given as illustrations with no limiting nature.

## EXAMPLES

Infrared spectra were recorded on a Nicolet 6700 FT-IR spectrophotometer.

**NMR measurements:** $^1H$ and $^{31}P$ MAS NMR spectra were recorded on a Bruker AVANCE-500 spectrometer (Larmor frequencies of 500.135, 202.461, and 125.768 MHz, respectively) at room temperature (304 K) using a 3.2 mm MAS probe. The following conditions were used for recording the one-dimensional (1D) $^1H$ MAS NMR spectra: 90° $^1H$ pulse = 1.8 μs; delay time between scans = 1 s. 16 scans were collected for each 1D $^1H$ MAS NMR spectrum. $^{31}P$ MAS NMR spectra with high power proton decoupling were recorded with or without cross-polarization (CP). The following conditions were used for recording the spectrum with CP in the two-dimensional (2D) $^{31}P\{^1H\}$ CPMAS heteronuclear correlation (HETCOR) NMR experiment: the proton r.f. field strength for decoupling was 45 kHz; contact time was 2 msec at the Hartmann-Hahn match condition of 53 kHz; the delay time between scans was 1 sec. The direct polarization (DP) $^{31}P\{^1H\}$ MAS NMR spectra were recorded with 90° flip angle pulses of 2.2 μsec duration and 21 sec recycle delay, which satisfied the $5 \times T_1$ condition. In these experiments, high power proton decoupling (75 kHz) was used only during the acquisition time. 64 scans were collected for each 1D $^{31}P\{^1H\}$ MAS NMR spectrum. For the 2D $^{31}P\{^1H\}$ CPMAS HETCOR NMR experiment a total of 64 $t_1$ increments with 256 scans each were collected. The $^{31}P\{^1H\}$-$^{31}P\{^1H\}$ DQ-SQ MAS spectrum was obtained using the POSTC7 sequence with excitation and conversion periods of 0.8 msec. The 2D spectra were collected with 128 $t_1$ increments of 50 μs and 16 transients each using the hypercomplex method. Presaturation pulses prior to the first pulse of the 2D sequence were used to reduce the recycle delay to 10 sec without saturation. The spin rate was 20 kHz for 1D 1H MAS, 1D $^{31}P\{^1H\}$ MAS, and 2D $^{31}P\{^1H\}$ CPMAS HETCOR, and 10 kHz for 2D $^{31}P\{^1H\}$-$^{31}P\{^1H\}$ DQ-SQ MAS. $^1H$ chemical shifts were referenced with respect to external TMS, while $^{31}P$ chemical shifts were referenced with respect to 85% $H_3PO_4$, with an accuracy of ± 0.4 ppm.

**Example 1:** preparation of $Rb(NH_4)_4[(Mo_2O_6)_2(O_3PC(CH_2C_3H_4N_2)(O)PO_3)_2Mn]._{14}H_2O$ **($Mo_4Zol_2Mn$)**.

To a solution of $Na_2MoO_4{\cdot}2H_2O$ (0.242 g, 1 mmol) in 10 mL of 1M $CH_3COONH_4/CH_3COOH$ buffer was added $Mn(OAc)_3{\cdot}2H_2O$ (0.070 g, 0.26 mmol) and zoledronic acid (0.137 g, 0.5 mmol). The solution was stirred for 5 min then $NH_3$ (33% in water) was added dropwise to pH = 7.5. The solution was left to evaporate and was filtered after 24 h in order to remove a pink precipitate. EDX measurements and IR spectroscopy indicated that the precipitate was a manganese BP complex that did not contain molybdenum. Greyish crystals of the title compound appeared after three days.

Yield: 0.058 g (16% based on Mo). Anal. Calc. (found) for $C_{10}H_{56}MnMo_4N_{8.3}O_40P_4Rb_{0.7}$ (M.W. = 1555 g mol-1): C 7.72 (7.71), H 3.63 (3.58), N 7.47 (7.25), Mn 3.53 (3.60), Mo 24.67 (24.52), P 7.97 (7.90), Rb 3.85 (3.42). IR (FTR): ν (cm-1) = 1576(m), 1544 (w), 1422(s), 1290 (w), 1138(s), 1109(sh), 1045(s), 976(m), 912(s), 873(s), 785(s), 696(m), 656(m), 619(w), 560(w), 528(m).

**Example 2**: preparation of $(NH_4)_{5[}(Mo_2O_6)_2(O_3PC(C_3H_6NH_3)(O)PO_3)_2Fe].11H_2O$ **($Mo_4Ale_2Fe$)**

To a solution of $(NH_4)_6Mo_7O_{24}{\cdot}4H_2O$ (0.989 g, 0.80 mmol) in 10 mL of water was added $H_5Ale$ (0.697 g, 2.80 mmol) and $FeCl_3{\cdot}6H_2O$ (0.378 g, 1.4 mmol). The solution was stirred for 5 min then $NH_3$ (33% in water) was added dropwise to pH = 6.0. The solution was then stirred for 40 min at 90 °C until clear. After cooling to room temperature, a fine powder was filtered off and the solution left to evaporate. Yellow crystals appeared after 30 min and were collected after two days.

Yield: 1.20 g (61% based on Mo). Anal. Calc. (found) for $C_8H_{60}FeMo_4N_7O_{37}P_4$ (M.W. = 1410 g $mol^{-1}$): C 6.81 (6.89), H 4.29 (4.15), N 6.95 (6.99), P 8.79 (8.68), Mo 27.21 (26.99), Fe 3.96 (3.94). IR (FTR): ν ($cm^{-1}$) = 1602(sh), 1421(vs), 1130(s), 1039(sh), 858(s), 802(s), 690(w), 641(m), 574(w), 510(m), 481(vw).

**<u>Example 3</u>:** preparation of $(NH_4)_5[(Mo_2O_6)_2(O_3PC(C_7H_{14}NH_3)(O)PO_3)_2Fe].10H_2O$ **($Mo_4(BPC_8NH_2)_2Fe$)**

A mixture of $(NH_4)_6Mo_7O_{24}\cdot 4H_2O$ (0.200 g, 0.16 mmol), $FeCl_3.6H_2O$ (70 mg, 0.25 mmol) and $H_5(BPC_8NH_2)$ (0.175 g, 0.57 mmol) in 20 mL of water was stirred and the pH adjusted to 7.5 by addition of $NH_3$ (33% solution). The mixture was heated to 90°C for 1 h and then cooled to room temperature. The resulting mixture was filtered and the filtrate left to slowly evaporate. After two weeks, yellow crystals were collected by filtration.

Yield: 0.145 g (34% based on Mo). Anal. Calc. (found) for $C_{16}H_{74}FeMo_4N_7O_{36}P_4$ (M.W. = 1504 g $mol^{-1}$): C 12.77 (12.71), H 4.95 (4.66), N 6.51 (6.17), Fe 3.71 (3.70), Mo 25.51 (25.82), P 8.24 (8.38). IR (FTR): ν ($cm^{-1}$) = 1615(sh), 1417(s), 1117(s), 1041(vs), 1018(vs), 940(w), 905(s), 860(s), 789(s), 702(s), 653(s), 573(s), 513(s), 484(s).

**<u>Example 4</u>:** preparation of $Na_{0.s}(NH_4)_{6.5}[(Mo_2O_6)_2(O_3PC(C_8H_{17})(O)PO_3)_2Fe].10H_2O$ **($Mo_4(BPC_9)_2Fe$)**

A mixture of $(NH_4)_6Mo_7O_{24}.4H_2O$ (0.248 g, 0.20 mmol), $FeCl_3.6H_2O$ (0.095 g, 0.35 mmol) and $Na_2H_3(BPC_9)$ (0.258 g, 0.74 mmol) in 5 mL of 1 M $CH_3COONH_4/CH_3COOH$ buffer was stirred and the pH adjusted to 7.5 by addition of $NH_3$ (33% solution). The mixture was then sealed in a 23 mL Teflon-lined stainless steel reactor and heated to 130°C over a period of 4 h, kept at 130°C for 20 h, then cooled to room temperature over a period of 36 h. The resulting mixture was filtered and the filtrate left to slowly evaporate. After five days, yellow crystals were collected by filtration.

Yield: 0.075 g (53% based on Mo). Anal. Calc. (found) for $Na_{0.5}C_{18}H_{81}FeMo_4N_{6.5}O_{36}P_4$ (M.W. = 1540 g $mol^{-1}$): C 14.04 (13.98), H 5.30 (5.38), N 5.91 (6.22), Fe 3.63 (3.61), Mo 24.92 (25.08), P 8.05 (8.01), Na 0.75 (0.65). IR (FTR) : ν ($cm^{-1}$) = 1635(w), 1419(s), 1115(m), 1036(s), 1009(s), 913(s), 892(s), 860(m), 794(s), 694(s), 667(w), 650(s), 571(vs), 509(s), 483(s).

**<u>Example 5</u>:** preparation of $(NH_4)_5[(Mo_2O_6)_2(O_3PC(CH_2C_3H_4N_2)(O)PO_3)_2Fe].5H_2O$ **($Mo_4Zol_2Fe$)**

A mixture of $(NH_4)_6Mo_7O_{24}.4H_2O$ (0.248 g, 0.20 mmol), $FeCl_3.6H_2O$ (0.095 g, 0.35 mmol) and zoledronic acid (0.201 g, 0.77 mmol) in 5 mL of 1 M $CH_3COONH_4/CH_3COOH$ buffer was stirred and the pH adjusted to 6 by addition of $NH_3$ (33% solution). The mixture was sealed in a 23 mL Teflon-lined stainless steel reactor and heated to 130°C over a period of 4 h, kept at 130°C for 20 h, then cooled to room temperature over a period of 36 h. Yellow crystals were collected by filtration and were washed with water.

Yield: 0.315 g (67% based on Mo). Anal. Calc. (found) for $C_{10}H_{42}FeMo_4N_9O_{31}P_4$ (M.W. = 1348 g $mol^{-1}$): C 8.92 (8.87), H 3.14 (3.09), N 9.35 (9.36), Mo 28.46 (28.36), Fe 4.14 (4.21), P 9.19 (9.20). IR (FTR) : ν ($cm^{-1}$) = 1652(w), 1575(m), 1545(m), 1429(vs), 1394(s), 1313(w), 1284(m), 1138(s), 1118(s), 1042(vs), 977(s), 948(m), 914(m), 869(s), 783(s), 708(s), 676(s), 621(s), 576(s), 516(s), 481(s).

**<u>Example 6:</u>** preparation of $Na_8\{Pt_4Cl_8(Mo_3O_8)_4[O_3PCC_3H_6NH_2CH_2(C_5H_4N)(O)PO_3]_4\}\cdot 30H_2O$ **($Mo_{12}(AlePy)_4Pt_4$)**

<u>Example 6.1</u>: synthesis of intermediate: $Na_3\{Na(Mo_3O_8)_4[O_3PCC_3H_6NH_2CH_2(C_5H_5N)(O)PO_3]_4\}$ .25H20 **($Mo_{12}(AlePy)_4$)**

To a solution of $Na_2MoO_4.2H_2O$ (0.582 g, 2.4 mmol) in 40 mL of water was added $Na_2H_3$(AlePy) (0.308 g, 0.70 mmol) and the pH was adjusted to 3 using a 2 M solution of HCl. The solution was stirred for 1 h at 90°C, cooled to room temperature and slowly evaporated. White crystals (0.240 g, yield 34% based on Mo) were filtered off after 3 days. Anal. Calc. (found) for $C_{40}H_{110}Mo_{12}N_8Na_4O_{85}P_8$ (M.W. = 3554 g $mol^{-1}$): C 13.52 (13.11), H 3.12 (3.02), Mo 32.39 (32.41), N 3.15 (3.01), Na 2.59 (2.88), P 6.88 (7.04). IR (FTR): ν ($cm^{-1}$) = 1623(m), 1529(w), 1451(m), 1145(sh), 1126(s), 1056(sh), 1026(s), 965(w), 917(m), 874(s), 794(m), 760(m), 682(sh), 647(m), 541(m), 520(m), 489(m), 401(m).

<u>Example 6.2</u>: preparation of $Na_8\{Pt4Cl_8(Mo_3O_8)_4[O_3PCC_3H_6NH_2CH_2(C_5H_4N)(O)PO_3]_4\}\cdot 30H_2O$ (**$Mo_{12}$(AlePy)$_4$Pt$_4$**)

To a suspension **of $Mo_{12}(AlePy)_4$** (0.12 g, 0.033 mmol) in 3 mL of water was added a solution of $Pt(DMSO)_2Cl_2$ (0.078 g, 0.185 mmol) in 2 mL of water heated to 60°C and the pH was adjusted to 3 using 2 M HCl. The solution was stirred for 1 h at 90 °C, cooled to room temperature and NaCl (0.500 g, 8.55 mmol) added. The solution was stirred for 1 h. A beige precipitate was collected by filtration (0.055 g, yield 35% based on Mo). Anal. Calc. (found) for $C_{40}H_{116}Cl_8Mo_{12}N_8Na_8O_{90}P_8Pt_4$ (M.W. = 4796 g $mol^{-1}$): C 10.02 (11.02), H 2.44 (2.45), Cl 5.91 (3.55), Mo 24.00 (23.42), N 2.33 (2.33), Na 3.83 (4.04), P 5.17 (5.26), Pt 16.27 (16.00). IR (FTR) : ν ($cm^{-1}$) = 1612(m), 1489(w), 1425(m), 1121(s), 1024(s), 922(sh), 883(s), 789(m), 767(m), 676(sh), 652(m), 545(m).

Example 6.3: determination of the structure of **$Mo_{12}(AlePy)_4Pt_4$**

**Solid State NMR Spectroscopy**

Since it was not possible to determine the structure of the **$Mo_{12}(AlePy)_4Pt_4$** complex by using crystallography, it was determined by the comparison of the solid state NMR spectra of **$Mo_{12}(AlePy)_4$** (which is essentially insoluble in water) and **$Mo_{12}(AlePy)_4Pt_4$** using one and two-dimensional magic-angle sample spinning techniques. The 1D $^{31}P\{^1H\}$ MAS NMR spectrum of **$Mo_{12}(AlePy)_4$** exhibits four resolved lines, at 18.1 (23%), 20.0 (23%), 25.3 (27%), and 26.4 ppm (27%), in agreement with the four crystallographic P sites. The 2D $^{31}P\{^1H\}$-$^{31}P\{^1H\}$ DQ-SQ correlation experiment shows strong dipolar coupling between the resonances at 18.1 and 26.4 ppm, as well as those at 20.0 and 25.3 ppm, consistent with two distinct **AlePy** environments, similar to the observation of two pairs of signals in the range 26-27 and 29-33 ppm observed for alendronate in $[(Mo_2O_2X_2(H_2O))_4(Ale)_4]^{8-}$, where X = O or S (H. El Moll et al Dalton Trans. 2012, 41, 9955). The MAS NMR spectrum of the platinum complex **$Mo_{12}(AlePy)_4Pt_4$** shows two signals, centered at ca. 21 and 25 ppm, about the same as observed with **$Mo_{12}(AlePy)_4$,** but with obvious line-broadening. The broadening could be due either to the low crystallinity in **$Mo_{12}(AlePy)_4Pt_4$** and/or, a dynamic disorder, since we also observe a much less efficient CP transfer in CPMAS experiments when compared with **$Mo_{12}(AlePy)_4$.**

The $^1H$ MAS spectrum of **$Mo_{12}(AlePy)_4$** consists of a dominant resonance at 4.2 ppm due to water molecules, with a very small shoulder at ca. 2 ppm, and two other signals, centred at ~ 8 and 18.4 ppm. The resonances around 2 and 8 ppm are assigned to methylene and aromatic protons of the **AlePy** ligands. The signal at ca. 18 ppm arises from acidic protons and the results of a 2D $^{31}P\{^1H\}$ HETCOR experiment clearly indicates close proximity between this/these proton/s and the phosphorus responsible for the resonance at 20 ppm. A close inspection of the structure indicates that of the four phosphonate groups (PI, P2, P3 and P4) only one, P2, is involved in a strong hydrogen bond interaction, via its terminal P=O group (Figure 2). More specifically, P2 has a 1.7 Å intermolecular N-H···O=P hydrogen bond between the protonated pyridine group (N24-H24) and the terminal oxygen (029) of the phosphonate P2 in the crystal structure. From these observations the $^{31}P$ NMR signals was thus assigned as follows: P1/P2=25 ppm/20 ppm and P3/P4=18 ppm/26 ppm, where P1 and P4 correspond to $(P-(OMo)_3)$ and P2 and P3 correspond to $(O=P-(OMo)_2)$. The signal at 18.4 ppm in the $^1H$ spectrum thus corresponds to the pyridinium proton of the **AlePy** ligand. The $^1H$ MAS spectrum of the Pt complex **$Mo_{12}(AlePy)_4Pt_4$** is very similar to that of **$Mo_{12}(AlePy)_4$,** the only difference being the absence of the resonance at 18.4 ppm, clearly, a consequence of the complexation of the pyridine to Pt.

**Example 7: X-ray crystallography measurement**

Data collection was carried out by using a Siemens SMART three-circle diffractometer for **$Mo_4Ale_2Fe$** and **$Mo_4(BPC_9)_2Fe$** and by using a Bruker Nonius X8 APEX 2 diffractometer for **$Mo_2Ale_2Mn$, $Mo_4(BPC_8NH_2)_2Fe$** and **$Mo_{12}(AlePy)_4$.** Both were equipped with a CCD bi-dimensional detector using the monochromatized wavelength $\lambda$(Mo K$\alpha$) = 0.71073 Å. Absorption correction was based on multiple and symmetry-equivalent reflections in the data set using the SADABS program (G. M. Sheldrick, SADABS; program for scaling and correction of area detector data, University of Göttingen, Germany, 1997) based on the method of Blessing (R. Blessing, Acta Crystallogr. 1995, A51, 33). The structures were solved by direct methods and refined by full-matrix least-squares using the SHELX-TL package (G. M. Sheldrick, SHELX-TL version 5.03, Software Package for the Crystal Structure Determination, Siemens Analytical X-ray Instrument Division : Madison, WI USA, 1994). In all structures there are small discrepancies between the formulae determined by elemental analysis and those deduced from the crystallographic atom list because of the difficulty in locating all disordered water molecules (and sometimes the counter-cations), a common feature found in other polyoxometalate structural investigations (See for example a) M. Sadakane, M. H. Dickman, M. T. Pope, Angew. Chem. Int. Ed. 2000, 39, 2914; b) C. Zhang, R. C. Howell, K. B. Scotland, F. G. Perez, L. Torado, L. C. Francesconi, Inorg. Chem. 2004, 43, 7691; c) Z. Zhang, Y. Qi, C. Qin, Y. Li, E. Wang, X. Wang, Z. Su, L. Xu, Inorg. Chem. 2007, 46, 8162; d) N. Belai, M. T. Pope, Chem. Common. 2005, 46, 5760). In the structures of **$Mo_4Ale_2Fe$** and **$Mo_4(BPC_9)_2Fe$,** $NH_4^+$ and $H_2O$ could not be distinguished based on the observed electron densities; therefore, all the positions were labelled O and assigned the oxygen atomic diffusion factor. Crystallographic data are given in **Table 2.**

**Table 2**

| | $Mo_4Ale_2Mn$ | $Mo_4Ale_2Fe$ | $Mo_4(BPC_8NH_2)_2Fe$ | $Mo_4(BPC_9)_2Fe$ | $Mo_4Zol_2Fe$ | $Mo_{12}(AlePy)_4$ |
|---|---|---|---|---|---|---|
| Empirical formula | $C_8H_{56}MnMo_4N_{6.50}Na_{0.50}O_{35}P_4$ | $C_8H_{60}FeMo_4N_7O_{37}P_4$ | $C_{16}H_{82}FeMo_4N_7O_{36}P_4$ | $C_{18}H_{81}FeMo_4Na_{0.5}N_{6.5}O_{36}P_4$ | $C_{10}H_{42}FeMo_4N_9O_{31}P_4$ | $C_{40}H_{110}Mo_{12}N_8Na_4O_{85}P_8$ |
| Formula weight [g] | 1377.60 | 1410.12 | 1512.35 | 1539.88 | 1348.02 | 3554.32 |
| Temperature [K] | 200 | 293 | 200 | 293 | 149 | 293 |
| Crystal system | Monoclinic | Monoclinic | Monoclinic | Monoclinic | Triclinic | Orthorhombic |
| Space group | $P2_1/n$ | $P2_1/n$ | $P2_1/c$ | $P2_1/n$ | P-1 | $P2_12_12$ |
| $a$ [Å] | 10.860(1) | 10.798(4) | 12.8986(3) | 8.538(9) | 10.003(1) | 17.472(4) |
| $b$ [Å] | 17.096(2) | 17.340(6) | 23.6753(5) | 27.89(13) | 12.9246(9) | 21.053(4) |
| $c$ [Å] | 11.885(1) | 11.799(4) | 8.4687(2) | 23.92(2) | 16.529(1) | 15.655(4) |
| $\alpha$ [°] | 90 | 90 | 90 | 90 | 78.132(2) | 90 |
| $\beta$ [°] | 97.055(4) | 98.271(7) | 107.583(1) | 92.35(2) | 74.463(2) | 90 |
| $\gamma$ [°] | 90 | 90 | 90 | 90 | 81.480(2) | 90 |
| $V$ [$Å^3$] | 2189.9(4) | 2182.7(14) | 2465.3(1) | 5707(10) | 2005.1(3) | 5759(2) |
| $Z$ | 2 | 2 | 2 | 4 | 2 | 2 |
| $\rho_{calc}$ [g $cm^{-3}$] | 2.095 | 2.146 | 2.026 | 1.792 | 2.233 | 1.863 |
| $\mu$ [$mm^{-1}$] | 1.653 | 1.704 | 1.514 | 1.314 | 1.841 | 1.474 |
| Data / Parameters | 6397/284 | 3139/267 | 7159/305 | 16250/637 | 6851/624 | 16652/635 |
| $R_{int}$ | 0.0231 | 0.0558 | 0.0517 | 0.0897 | 0.0341 | 0.0427 |
| GOF | 1.083 | 1.052 | 1.301 | 1.081 | 1.039 | 1.076 |
| $R_1$ (>2σ(I)) | 0.0331 | 0.0503 | 0.0602 | 0.0587 | 0.0402 | 0.0452 |

**Example 8: Magnetic studies**

Magnetic susceptibility measurements were carried out with a Quantum Design SQUID Magnetometer with an applied field of 1000 Oe for **$Mo_4Ale_2Mn$** and 10000 Oe for **$Mo_4Zol_2Mn$** using powder samples pressed into pellets to magnetic ordering of the crystallites. The independence of the susceptibility value with regard to the applied field was checked at room temperature. The susceptibility data were corrected for the diamagnetic contributions as deduced by using Pascal's constant tables. All the magnetic data have been simulated using a FORTRAN program.

Magnetic susceptibility measurements were carried out to deduce the Mn oxidation state in the **$Mo_4Ale_2Mn$** and **$Mo_4Zol_2Mn$** complexes, the latter being of particular interest since, as discussed below, it had the most potent activity in tumor cell growth inhibition. The $\chi_M T$ = f($T$) curve for **$Mo_4Ale_2Mn$** is shown in Figure 3a. The $\chi_M T$ product is constant in the 250 - 30 K temperature range, with a $\chi_M T$ value of 4.235 $cm^3$ $mol^{-1}$ K at 250 K, the theoretical $\chi_M T$ value being 4.375 $cm^3$ $mol^{-1}$ K for a mononuclear high-spin $Mn^{II}$ complex ($S$ = 5/2), assuming $g$ = 2.0. Beyond 30 K, due to single ion-anisotropy, the $\chi_M T$ curve continuously decreases, reaching 3.875 $cm^3$ $mol^{-1}$ K at 2 K. The magnetization was thus measured at 3, 4, 6 and 8 K as a function of the magnetic field. As can shown in Figure 3b, the data can be well fit by using $D$ = +0.97 $cm^{-1}$ and g = 1.97 in the spin Hamiltonian:

$$\hat{H} = g\beta H\hat{S} - D(\widehat{S_z^2} - \tfrac{1}{3}\hat{S}(\hat{S}+1))$$

where $\beta$ is the Bohr magneton, H the magnetic field, g the spectroscopic splitting factor and $D$ the axial zero-field splitting parameter (R = 1.4 $10^{-4}$)(R = [$\Sigma(M_{cal}\text{-}M_{obs})^2/\Sigma(M_{obs})^2$]). The |$D$| value is higher than those usually determined with six-coordinated $Mn^{II}$ complexes (for example C. Duboc et al. Chem. Eur. J. 2008, 14, 6498). However, it is known that polyoxometalate ligands can induce a strong magnetic anisotropy, as exemplified by the $D$ value of +1.46 $cm^{-1}$ found in the $Mn^{II}$ complex $[(As_2W_{20}O_{68})(Mn(H_2O))]^{8-}$(C. Pichon et al. Inorg. Chem. 2007, 46, 7710). The $\chi_M T$ = f($T$) curve for **$Mo_4Zol_2Mn$** Figure 3a again shows that the $\chi_M T$ product is constant in the 250 - 30 K temperature range but with a $\chi_M T$ value of 3.05 $cm^3$ $mol^{-1}$ K. This value is in good agreement with the theoretical $\chi_M T$ value of 3.00 $cm^3$ $mol^{-1}$ K calculated considering g = 2.0 for a mononuclear $S$ = 2 $Mn^{III}$ complex. Fits of the $M$ = f($H$) curves (Figure 3c) at $T$ = 3, 4, 6 and 8 K lead to $D$ = +3.20 $cm^{-1}$ and g = 2.02 (R = 4.6 $10^{-4}$).[34] Both the $D$ and g values fall within the range of those determined for mononuclear $Mn^{III}$ complexes(for example C. Duboc et al., J. Phys. Chem. A. 2010, 114, 10750). Also, the $\chi_M T$ = f($T$) curves of the **$Mo_4Ale_2Mn$** and **$Mo_4Zol_2Mn$** complexes can be fitted (Figure 3a) using the parameters deduced from the magnetization data (R = 3.2 $10^{-5}$ and 1.21 $10^{-4}$, respectively). These results are thus in agreement with the bond valence sum calculations noted above, confirming that **$Mo_4Ale_2Mn$** contains a $Mn^{II}$ center while **$Mo_4Zol_2Mn$** contains a $Mn^{III}$.

**Example 9: Biological data**

Example 9.1: Cell-growth inhibition assays

Human tumor cell lines MCF-7 (breast adenocarcinoma were obtained from the National Cancer Institute and maintained at 100% humidity and 5% $CO_2$ at 37 °C. Cell lines were cultured in RPMI-1640 medium supplemented with 2 mM L-glutamine and 10% fetal bovine serum (Gibco, Grand Island, NY) at 37°C in a 5% $CO_2$ atmosphere with 100% humidity. Compound stock solutions were typically prepared in water at a concentration of 0.04 M. A broth microdilution method was used to determine the bisphosphonate growth inhibition $IC_{50}$ values. Compounds were half log serial diluted using cell culture media into 96-well TC-treated round bottom plates (Corning Inc., Corning, NY) typically from 1264 μM to 40 nM, but in some cases compounds were run over a larger concentration range to enable accurate IC50 determinations. Cells were plated at a density of 5000 cells/well. Cells were then incubated under the same culture conditions for 4 days at which time an MTT ((3-(4,5-dimethylthi-azole-2-yl)-2,5-diphenyltetrazolium bromide) cell proliferation assay (ATCC, Manassas, VA) was performed to obtain dose response curves. Briefly, cells were incubated for 2 h under culture conditions with the tetrazolium salt, lysed with detergent, incubated overnight, protected from light at room temperature. The absorbance at 600 nm was read the following day using a SpectraMax Plus 384 spectrophotometer (Molecular Devices, Sunnyvale, CA). The compound concentration for 50% growth inhibition values ($IC_{50}$) were obtained by fitting absorbance data to a rectangular hyperbolic function: $I = (I_{max})(C)/IC_{50} + C$ where $I$ is the percent inhibition, $I_{max}$ = 100% inhibition and $C$ is the concentration of the inhibitor, using GraphPad PRISM 3.0 software for windows (Graphpad Software Inc., San Diego, CA).

Results are gathered in table 3 hereinafter. The results are shown on a molar basis ($IC_{50}$ values, in μM) as well as on a per-bisphosphonate basis, which is more logical when comparisons are to be made with the pure bisphosphonates.

Table 3

| Formula | $IC_{50}$(μM) | $IC_{50}$(μM) per BP |
|---|---|---|
| **$Mo_4Ale_2Mn$** | 5.7 ± 1.4 | 11 ± 2.8 |
| **$Mo_4Zol_2Mn$** | 1.3 ± 0.24 | 2.6 ± 0.48 |
| **Ale** | 210 ± 6.0 | 210 ± 6.0 |
| **Zol** | 15 ± 2.6 | 15 ± 2.6 |
| **$MnCl_2$** | 740 ± 140 | n/a |

Both the heteroatom as well as the bisphosphonate are having effects on cell growth inhibition, although addition of Mn(II)$Cl_2$ alone has essentially no effect ($IC_{50}$~740 μM) on cell growth.

Such results may be compared to the results as obtained in Hani El Moll et al., "polyoxometalates functionalized by bisphosphonate ligands: synthesis, structural, magnetic, and spectroscopic characterizations and activity on tumor cell lines", Inorg. Chem. 2012, 51, 7921-7931. Indeed said document discloses the same activity for polyoxometalates functionalized by bisphosphonate ligands which do not bear heteroatoms. The results are reproduced in the following table 4.

Table 4

| Formula | $IC_{50}$ (μM) | $IC_{50}$ (μM) per BP |
|---|---|---|
| **$Mo_6(Ale)_2$** | 56 ± 0.0 | 112 |
| **$Mo_6(Zol)_2$** | 2.2 ± 0.1 | 4.4 |
| **Ale** | 130 ± 2.2 | 130 |
| **Zol** | 7.7 ± 2.6 | 7.7 |

It may firstly be noted that structures with and without heteroatom may not be strictly compared insofar as the presence of the heteroatom influences the configuration of the complex. Moreover, the $IC_{50}$ values coming from the hereabove cited article may not be strictly compared because the measurement conditions are not strictly identical. However, trends may be stated with respect to the impact of the presence of the heteroatom within the structure.

Indeed, although the oxidation state in both compounds are different, the benefit of adding an heteroelement is clearly visible for the tested compounds ($Mo_4Ale_2Mn$ is far better than $Mo_6(Ale)_2$, $Mo_4Zol_2Mn$ is slightly better than $Mo_6(Zol)_2$).

Example 9.2: evidence for significant targeting of protein prenylation pathways

Addition of geranylgeraniol (GGOH) was tested on $Mo_4Zol_2Mn$.

Protocol Cell growth "rescue" experiments with geranylgeraniol were carried out as described previously, and namely in Y. Zhang, et al J. Am. Chem. Soc. 2009, 131, 51.

An increase in the $IC_{50}$ indicates a direct role for the bisphosphonate moiety in cell growth inhibition.

Results are gathered in table 5 hereinafter.

Table 5

| Formula | $IC_{50}$ (μM) | $IC_{50}$ (μM) +GGOH |
|---|---|---|
| **Zol** | 15 | 48 |
| **$Mo_4Zol_2Mn$** | 1.3 | 7.6 |

There is a major rescue with geranylgeraniol, just as found with zoledronate acting alone. These results indicate that there may be a significant contribution to cell growth inhibition from the presence of $Mn^{III}$.

The present invention is also related to the use of at least a compound chosen among a compound of any one of formula (I), (Ia) or (Ib) as defined above, and compounds (1) to (13) as defined above, for the manufacture of a pharmaceutical composition intended for the treatment of cancer.

The present invention also encompasses pharmaceutical compositions comprising at least a compound selected from the complexes of formulae (Ia) with the exclusion of complexes $Mo_4O_{12}Eti_2Mn$ (Mn(II)), $Mo_4O_{12}Ale_2Mn$ (Mn(II)), $Mo_4O_{12}Ale_2V$ (V(IV)), $Mo_4O_{12}Eti_2Fe$ (Fe(III)) and $Mo_4O_{12}Eti_2V$ (V(IV)) and (Ib) as defined above and compounds

(1) to (4) and (6) to (13) as defined above or any pharmaceutically acceptable salt thereof.

Thus, these pharmaceutical compositions contain an effective amount of said compound, and one or more pharmaceutical excipients.

The aforementioned excipients are selected according to the dosage form and the desired mode of administration.

In this context they can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance.

The present invention further relates to a method of treatment of patients suffering from cancer, which comprises at least a step of administration to a patient suffering thereof of an effective amount of a compound of any one of formulae (I), (Ia) or (Ib) as defined above and (1) to (13).

## Claims

1. Polyoxomolybdate-bisphosphonate complex containing a bisphosphonate ligand, wherein it further contains a heterometallic ion different from molybdenum, for use in treating cancer.

2. A polyoxomolybdate-bisphosphonate complex as defined in claim 1, for use in treating cancer, having the following formula (I)

$$[Mo_xO_y(BP)_zM_tX_r]^{n-} \qquad (I)$$

wherein

x, y, z, t, r and n are independent integers,
$2 \leq x \leq 12$,
$y > x$, and y ranges from 4 to 40,
$z \leq x$, and z ranges from 2 to 8,
$t < x$ and t ranges from 1 to 10,
r ranges from 0 to 8,
n represents an integer ranging from 2 to 16,
M represents a 3d transition metal ion selected from Mn, Fe, Cu, Zn, V and Co or a 4d or 5d transition metal like Ru or Pt,
X represents an halogen atom or a water molecule, and
BP represents a bisphosphonate.

3. A polyoxomolybdate-bisphosphonate complex as defined in claim 1 or 2, for use in treating cancer, wherein the polyoxometalate-bisphosphonate complex contains $Mo_4$ or $Mo_{12}$ clusters and in particular a $Mo_4$ cluster.

4. A polyoxomolybdate-bisphosphonate complex as defined in anyone of the preceding claims, for use in treating cancer, wherein the bisphosphonate has the following formula $H_2O_3PC(OH)(R)PO_3H_2$ (A)
wherein R represents

- a $(C_1\text{-}C_8)$alkyl group, optionally substituted by a NR1R2 group, or
- a

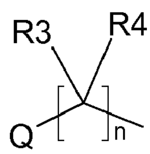

group wherein

n is 1, 2 or 3,
R3 and R4 independently of each other are selected from a hydrogen atom, a halogen, a $(C_1\text{-}C_4)$alkyl group

and a $(C_1-C_4)$alkoxy group, and
Q represents a phenyl group or a heteroaryl group, said phenyl and heteroaryl being optionally substituted with one to three substituents chosen from a $(C_1-C_{15})$alkyl group, a halogen atom, a hydroxyl group, an OR1 group, a SRI group, a NR1R2 group or a CN group, and
R1 and R2 independently represent a hydrogen atom, a $(C_1-C_6)$alkyl or a $-(CH_2)_xX$, wherein x is 1, 2 or 3, and X has the same meaning as Q as defined above.

**5.** A polyoxomolybdate-bisphosphonate complex as defined in the preceding claim, for use in treating cancer, wherein R represents a $(C_1-C_8)$alkyl group, optionally substituted by a NR1R2 group or a

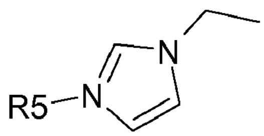

,

where R1 and R2 independently represent a hydrogen atom or a methylpyridine group, in particular a 2-methylpyridine group, and where R5 represents a hydrogen atom or a $(C_1-C_8)$alkyl group.

**6.** A polyoxomolybdate-bisphosphonate complex as defined in anyone of the preceding claims, for use in treating cancer, wherein the bisphosphonate ligand is chosen among alendronate, zolendronate, $BPC_8NH_2$, AlePy, BPH-1222 and $BPC_9$, and more particularly wherein M is Mn.

**7.** A polyoxomolybdate-bisphosphonate complex as defined in claim 4, for use in treating cancer, wherein the bisphosphonate ligand is defined as a compound of formula (A) wherein R represents a

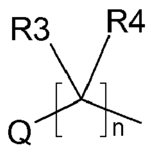

group in which n is 1, R3 and R4 are hydrogen atoms and Q is a

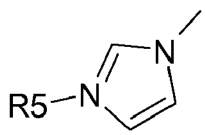

group, where R5 represents a hydrogen atom or a $(C_1-C_{15})$alkyl group.

**8.** A polyoxomolybdate-bisphosphonate complex as defined in anyone of the preceding claims, for use in treating cancer, wherein the heterometallic ion different from molybdenum is Mn.

**9.** A polyoxomolybdate-bisphosphonate complex as defined in anyone of the preceding claims, for use in treating cancer, wherein it is selected from (1) $Mo_4O_{12}Ale_2Fe$, (2) $Mo_4O_{12}$ $(BPC_8NH_2)_2Fe$, (3) $Mo_4O_{12}BPC_9)_2Fe$, (4) $Mo_4O_{12}Zol_2Fe$, (5) $Mo_4O_{12}Ale_2Mn$ (Mn(II)), (6) $Mo_4O_{12}Zol_2Mn$ (Mn(III)), (7) $Mo_{12}O_{32}(AlePy)_4Pt_4Cl_8$, (8) $Mo_4O_{12}Ale_2Mn$ (Mn(III)), (9) $Mo_4O_{12}Zol_2Mn$ (Mn(II)), (10) $Mo_4O_{12}(BPH\text{-}1222)_2Mn$ (Mn(II)), (11) $Mo_4O_{12}(BPH\text{-}1222)_2Mn$ (Mn(III)), (12) $Mo_4O_{12}Zol_2V$ (V(IV)) and (13) $Mo_4O_{12}Zol_2Ru$ (Ru(III)), and more particularly from $Mo_4O_{12}Ale_2Fe$, (2) $Mo_4O_dBPC_8NH_2)_2Fe$, (3) $Mo_4O_dBPC_9)_2Fe$, (4) $Mo_4O_{12}Zol_2Fe$, (5) $Mo_4O_{12}Ale_2Mn$ (Mn(II)), (6) $Mo_4O_{12}Zol_2Mn$ (Mn(III)) and (7) $Mo_{12}O_{32}(AleP_y)_4Pt_4Cl_8$.

**10.** A polyoxomolybdate-bisphosphonate complex for use in treating cancer, according to anyone of the preceding claim, wherein the cancer is selected from colorectal cancer, pancreatic cancer, lung cancer including non-small cell lung cancer, breast cancer, large intestine cancer, biliary tract cancer, bladder cancer, gall bladder cancer, thyroid cancer,

melanoma, liver cancer, uterine/cervical cancer, oesophageal cancer, kidney cancer, ovarian cancer, prostate cancer, head and neck cancer, and stomach cancer, and more particularly from lung, breast, large intestine, biliary tract and pancreatic cancer.

**11.** A polyoxomolybdate-bisphosphonate complex having the following formula (Ia)

$$[Mo_4O_{12}(BP)_2M]^{n-} \qquad (Ia)$$

wherein

BP is a bisphosphonate as defined in claim 4 or 5, and
M represents a 3d transition metal ion selected from Mn, Pt, Fe, Cu, Zn, V and Co or a 4d transition metal like Ru, and in particular M represents Mn, V or Ru, and more particularly Mn,
with the exclusion of compounds $Mo_4O_{12}Eti_2Mn$ (Mn(II)), $Mo_4O_{12}Ale_2Mn$ (Mn(II)), $Mo_4O_{12}Ale_2V$ (V(IV)), $Mo_4O_{12}Eti_2Fe$ (Fe(III)) and $Mo_4O_{12}Eti_2V$ (V(IV)) or

the following formula (Ib)

$$[Mo_{12}O_{32}(BP)_4M_4X_8]^{n-} \qquad (Ib)$$

wherein BP is a bisphosphonate as defined in anyone of claims 4 to 7,
X represents a halogen atom or a water molecule, and
M represents a 3d transition metal ion selected from Mn, Fe, Cu, Zn, V and Co or a 4d or 5d transition metal like Ru or Pt, and in particular M represents Mn, V or Ru, and more particularly Mn.

**12.** A polyoxomolybdate-bisphosphonate complex selected from $Mo_4O_{12}Ale_2Fe$ (1), $Mo_4O_{12}(BPC_8NH_2)_2Fe$ (2), $Mo_4O_{12}(BPC_9)_2Fe$ (3), $Mo_4O_{12}Zol_2Fe$ (4), $Mo_4O_{12}Zol_2Mn$ (Mn(III)) (6), $Mo_{12}O_{32}(AlePy)_4Pt_4Cl_8$ (7), $Mo_4O_{12}Ale_2Mn$ (Mn(III)) (8), $Mo_4O_{12}Zol_2Mn$ (Mn(II)) (9), $Mo_4O_{12}$BPH-1222$)_2$Mn (Mn(II)) (10), $Mo_4O_{12}$(BPH-1222$)_2$Mn (Mn(III)) (11), $Mo_4O_{12}Zol_2V$ (V(IV)) (12) and $Mo_4O_{12}Zol_2Ru$ (Ru(III)) (13), and more particularly selected from $Mo_4O_{12}Zol_2Mn$ (Mn(III)) and $Mo_4O_{12}Zol_2Mn$ (Mn(II)).

**13.** A pharmaceutical composition comprising a polyoxomolybdate as defined in anyone of claims 11 and 12.

**14.** A synthetic preparation process for obtaining a polyoxomolybdate as defined in anyone of claims 11 and 12, consisting in reacting

(i) a precursor selected from $Na_2MoO_4$ and $(NH_4)_6Mo_7O_{24}$ within a one-pot procedure in presence of a solvent, in particular water buffered between 3 and 8, BP, a M metal salt at a temperature ranging from 20 to 130°C, or
(ii) a $Na_2MoO_4$ precursor within a two step procedure comprising a first step using said $Na_2MoO_4$ precursor in presence of a solvent, in particular water buffered between 3 and 5, followed by a second step using the obtained $Mo_{12}(BP)_4$ in presence of a solvent, in particular water buffered between 3 and 5, BP, a M precursor at a temperature ranging from 20 to 100°C.

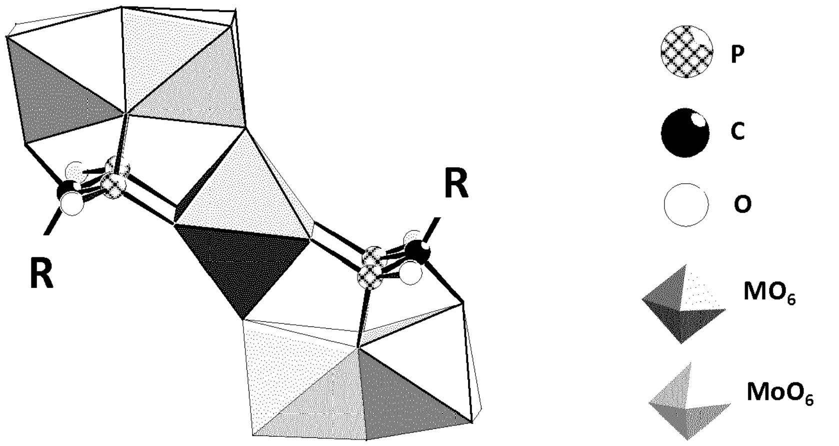

Figure 1

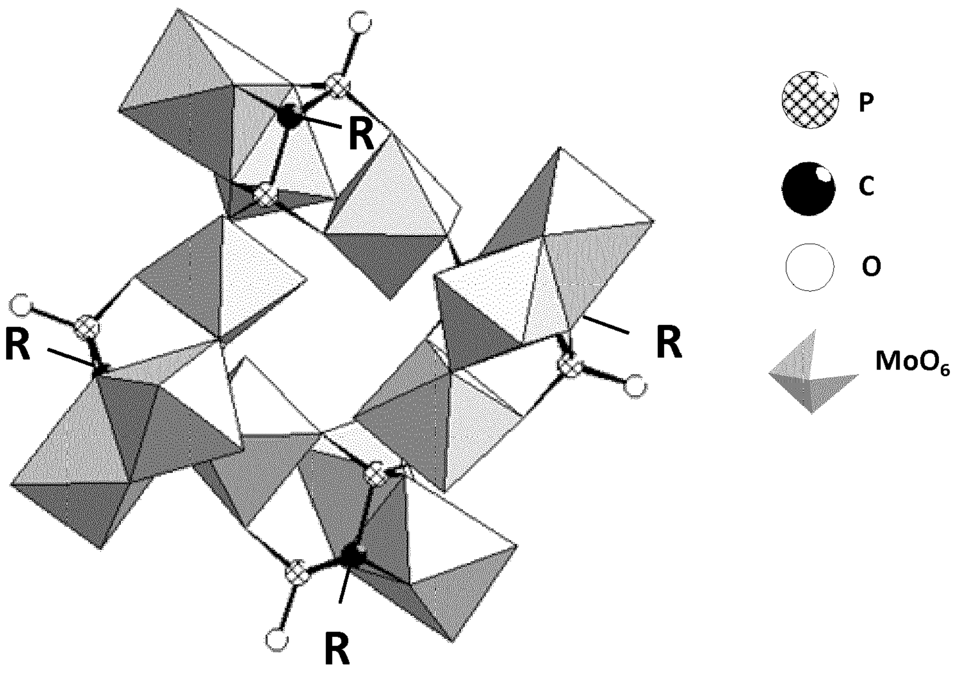

Figure 2

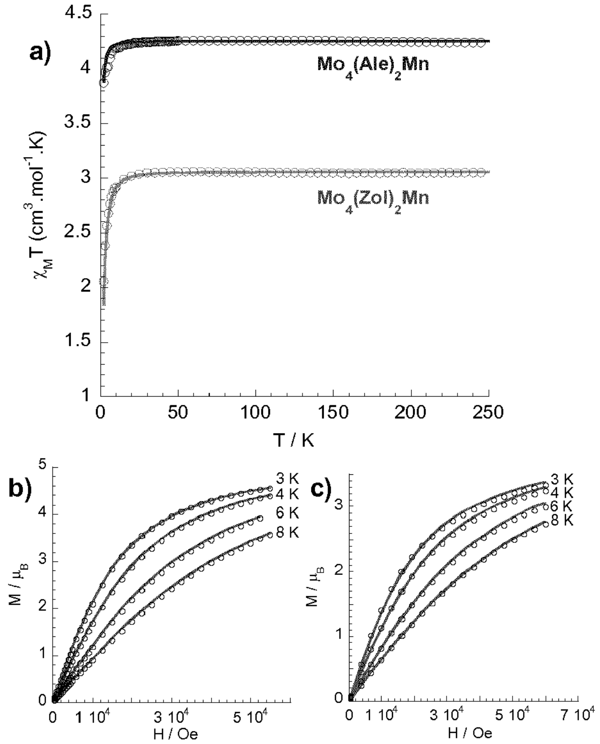
a)
4.5
4
3.5
3
2.5
2
1.5
1
$\chi_M T$ (cm$^3$.mol$^{-1}$.K)
Mo$_4$(Ale)$_2$Mn
Mo$_4$(Zol)$_2$Mn
0
50
100
150
200
250
T / K
b)
M / $\mu_B$
3 K
4 K
6 K
8 K
H / Oe
c)
M / $\mu_B$
3 K
4 K
6 K
8 K
H / Oe

**Figure 3**

Europäisches Patentamt
European Patent Office
Office européen des brevets

# EUROPEAN SEARCH REPORT

Application Number
EP 15 30 5812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEAN-DANIEL COMPAIN ET AL: "Tetra- to Dodecanuclear Oxomolybdate Complexes with Functionalized Bisphosphonate Ligands: Activity in Killing Tumor Cells", CHEMISTRY - A EUROPEAN JOURNAL., vol. 16, no. 46, 10 December 2010 (2010-12-10), pages 13741-13748, XP055225664, WEINHEIM, DE ISSN: 0947-6539, DOI: 10.1002/chem.201001626 | 1,10 | INV. C07F11/00 C07F13/00 C07F15/00 C07F15/02 C07F19/00 A61P35/00 A61K31/663 |
| Y | * tables 2 entries 2, 4 * * compounds 3,5 * | 2-9, 11-14 | |
| | ----- | | |
| X | HANI EL MOLL ET AL: "Polyoxometalates Functionalized by Bisphosphonate Ligands: Synthesis, Structural, Magnetic, and Spectroscopic Characterizations and Activity on Tumor Cell Lines", INORGANIC CHEMISTRY, vol. 51, no. 14, 16 July 2012 (2012-07-16), pages 7921-7931, XP055225639, EASTON, US ISSN: 0020-1669, DOI: 10.1021/ic3010079 | 1,10 | **TECHNICAL FIELDS SEARCHED (IPC)** |
| Y | * table 2 entry 3 * * compound Mo6(Sul)2 * | 2-9, 11-14 | C07F A61P A61K |
| | ----- | | |
| X | JINGYANG NIU ET AL: "Organodiphosphonate-Functionalized Lanthanopolyoxomolybdate Cages", CHEMISTRY - A EUROPEAN JOURNAL., vol. 18, no. 22, 29 May 2012 (2012-05-29), pages 6759-6762, XP055225656, WEINHEIM, DE ISSN: 0947-6539, DOI: 10.1002/chem.201200974 | 1-14 | |
| Y | * page 6759, column 1, line 4 * * page 6759, column 2, paragraph 2 * | 1-14 | |
| | ----- | | |
| | -/-- | | |

The present search report has been drawn up for all claims

1

| Place of search | Date of completion of the search | Examiner |
|---|---|---|
| The Hague | 25 November 2015 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 30 5812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAAD ALI ET AL: "Molybdenum Bisphosphonates with Cr(III) or Mn(III) Ions", JOURNAL OF CLUSTER SCIENCE, SPRINGER NEW YORK LLC, US, vol. 25, no. 3, 1 November 2013 (2013-11-01), pages 795-809, XP035335317, ISSN: 1040-7278, DOI: 10.1007/S10876-013-0655-3 [retrieved on 2013-11-01] | 1-14 | |
| Y | * page 796, line 17 - line 21 * * page 796, lines 28-30 * * abstract * * page 807, line 8 - line 10 * ----- | 1-14 | |
| X | HUAQIAO TAN ET AL: "Two new cantilever-type polyoxometalates constructed from {Mo 2 O 4 } 2+ fragments and diphosphonates", DALTON TRANSACTIONS: THE INTERNATIONAL JOURNAL FOR INORGANIC, ORGANOMETALLIC AND BIOINORGANIC CHEMISTRY, vol. 39, no. 5, 1 January 2010 (2010-01-01), pages 1245-1249, XP055229488, GB ISSN: 1477-9226, DOI: 10.1039/B912321K | 1-14 | TECHNICAL FIELDS SEARCHED (IPC) |
| Y | * page 1245, column 1, line 5 * * abstract * ----- -/-- | 1-14 | |

The present search report has been drawn up for all claims

1

| Place of search | Date of completion of the search | Examiner |
|---|---|---|
| The Hague | 25 November 2015 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 30 5812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br>Y | LIJUAN ZHANG ET AL: "A general synthesis approach in action: preparation and characterization of polyoxomolybdenum(vi) organophosphonates through oxidative Mo-Mo bond cleavage in {MoV2O4}", CRYSTENGCOMM, vol. 14, no. 14, 1 January 2012 (2012-01-01), page 4826, XP055229493, DOI: 10.1039/c2ce25093d<br>* abstract *<br>* page 4826, column 1, line 1 *<br>----- | 1-14<br><br><br><br><br><br><br><br><br><br>1-14 | |
| | | | TECHNICAL FIELDS SEARCHED (IPC) |

The present search report has been drawn up for all claims

1

| Place of search | Date of completion of the search | Examiner |
|---|---|---|
| The Hague | 25 November 2015 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **B. HASENKNOPF.** polyoxometalates: introduction to a class of inorganic compounds and their biomedical applications. *Frontiers in Bioscience,* 01 January 2005, vol. 10, 275-287
- **T. YUEN et al.** *Proc. Natl. Acad. Sci. USA,* 2014, vol. 11, 17989
- **A. STACHNIK et al.** *Proc. Natl. Acad. Sci. USA,* 2014, vol. 111, 17995
- **A. BANERJEE et al.** *Chem. Soc. Rev.,* 2012, vol. 41, 7590
- **A. DOLBECQ et al.** *Chem. Commun.,* 2012, vol. 48, 8299
- **L. YANG et al.** *Cryst. Growth. Des.,* 2013, vol. 13, 2540
- **H. EL MOLL et al.** polyoxometalales functionalized by bisphonate ligands: synthesis, structural, magnetic and spectroscopic characterizations and activity on tumor cell lines. *Inorg. Chem.,* 2012, vol. 51, 7921-7931
- *Inorg. Chem.,* 2012, vol. 51, 7921-7931
- **Y. LI ; E.WANG et al.** Two new cantilever-type polyoxometalates constructed from {Mo2O4}2+ fragments and diphosphonates. *Dalton Trans,* 2010, vol. 39, 1245
- **A. DOLBECQ et al.** Molybdenum bisphosphonates with Cr(III) or Mn(III) ions. *J. Clust. Sci.,* 2014, vol. 25, 795
- **Y. ZHOU et al.** A general synthesis approach in action: preparation and characterization of polyoxomolybdenum(VI) organophosphonates through oxidative Mo-Mo bond cleavage in {MoV2O. *CrystEng Comm,* 2012, vol. 14, 4826
- **A. SAAD et al.** *J. Clust. Sci.,* 2014, vol. 25, 795
- **YIFENG XIA et al.** a combination therapy for KRAS-driven lung adenocarcinomas using lipophilic bisphosphonates and rapamycin. *ScienceTranslationMedicine,* 2014, vol. 6 (263), 263ra161
- **MILLER et al.** *Frontiers in Genetics,* 2012, vol. 2, 1-9
- **A. DOLBECQ et al.** *J. Clust Sci.,* 2014, vol. 25, 795
- **H. EL MOLL et al.** *Dalton Trans.,* 2012, vol. 41, 9955
- **G. M. SHELDRICK.** *SADABS; program for scaling and correction of area detector data, University of Göttingen, Germany,* 1997
- **R. BLESSING.** *Acta Crystallogr.,* vol. A51, 33
- **G. M. SHELDRICK.** SHELX-TL version 5.03. *Software Package for the Crystal Structure Determination, Siemens Analytical X-ray Instrument Division : Madison, WI USA,* 1994
- **M. SADAKANE ; M. H. DICKMAN ; M. T. POPE.** *Angew. Chem. Int. Ed.,* 2000, vol. 39, 2914
- **C. ZHANG ; R. C. HOWELL ; K. B. SCOTLAND ; F. G. PEREZ ; L. TORADO ; L. C. FRANCESCONI.** *Inorg. Chem.,* 2004, vol. 43, 7691
- **Z. ZHANG ; Y. QI ; C. QIN ; Y. LI ; E. WANG ; X. WANG ; Z. SU ; L. XU.** *Inorg. Chem.,* 2005, vol. 46, 8162
- **N. BELAI ; M. T. POPE.** *Chem. Common.,* 2005, vol. 46, 5760
- **C. DUBOC et al.** *Chem. Eur. J.,* 2008, vol. 14, 6498
- **C. PICHON et al.** *Inorg. Chem.,* 2007, vol. 46, 7710
- **C. DUBOC et al.** *J. Phys. Chem. A.,* 2010, vol. 114, 10750
- **HANI EL MOL et al.** polyoxometalates functionalized by bisphosphonate ligands: synthesis, structural, magnetic, and spectroscopic characterizations and activity on tumor cell lines. *Inorg. Chem.,* 2012, vol. 51, 7921-7931
- **Y. ZHANG et al.** *J. Am. Chem. Soc.,* 2009, vol. 131, 51